Europäisches Patentamt

European Patent Office

Office européen des brevets

(19)

(11) Publication number : **0 479 518 A1**

## EUROPEAN PATENT APPLICATION

(12)

(21) Application number : **91308913.2**

(22) Date of filing : **30.09.91**

(51) Int. Cl.⁵ : **A61K 7/48, A61K 31/07**

(30) Priority : **01.10.90 GB 9021320**

(43) Date of publication of application :
**08.04.92 Bulletin 92/15**

(84) Designated Contracting States :
**AT BE CH DE DK ES FR GR IT LI LU NL SE**

(71) Applicant : **DIOMED DEVELOPMENTS LIMITED**
**Tatmore Place, Gosmore Nr. Hitchin**
**Hertfordshire, SG4 7QR (GB)**

(72) Inventor : **Whitefield, Martin**
**19 Parkway**
**London NW11 (GB)**

(74) Representative : **Collier, Jeremy Austin Grey et al**
**J.A.Kemp & Co., 14 South Square, Gray's Inn**
**London WC1R 5LX (GB)**

(54) **Compositions for topical application.**

(57)    Compositions for topical application for the treatment of acne comprise retinoic acid and optionally an antibiotic dissolved in an aqueous gel based on polyacrylic acid and having a pH near 7. Such compositions have improved cosmetic acceptability and clinical efficacy.

EP 0 479 518 A1

This invention relates to compositions for topical application, and more particularly to compositions containing retinoic acid.

All trans retinoic acid, i.e. 3,7-dimethyl-9-(2,6,6-trimethyl-1-cyclohexen-1-yl)-2,4,6,8-nonatetraenoic acid, also known as Vitamin A acid, and referred to herein simply as retinoic acid, has been used topically in the treatment of acne vulgaris for many years and more recently to reverse the effects of photo-aging of the skin, i.e. damage to the skin caused by prolonged exposure to the sun. In both clinical circumstances, the site most concerned is the face where it is particularly important to apply a formulation which, apart from being stable and effective, is also cosmetically acceptable on such a delicate area of skin. Retinoic acid is insoluble in water and, being chemically highly unsaturated, is very susceptible to degradation by atmospheric oxidation. For both these reasons it has hitherto only been formulated in solution in ethanol as a lotion or gel or as a suspension in a cream vehicle. The gel and lotion vehicles, although effective, are anhydrous and are therefore very drying and sometimes irritant to the skin, especially that of the face. The cream provides a much lower level of efficacy because the retinoic acid is inefficiently released into the skin.

In the case of acne, which is normally associated with excessive grease or sebum, there is some advantage in providing a drying alcoholic vehicle but, since retinoic acid itself is irritant, the drying effect of the alcohol exacerbates the irritant effect of the retinoic acid and many patients cannot tolerate the treatment. The cream formulation is not only less effective clinically but also involves use of a greasy vehicle which is inherently unsuitable for use in a dermatological condition for which excessive grease is a predisposing feature. In the case of photo-aged skin, the alcoholic formulations are generally too irritant and use of the cream is preferable, although the latter provides reduced effectiveness.

International Publication WO90/14833 describes aqueous gel compositions for topical application to the skin comprising an aqueous medium, a retinoid, which may be retinoic acid, a gelling agent effective to form a gel and hold the retinoid for slow release in the aqueous medium, and an antioxidant. The gelling agent is preferably polyacrylic acid neutralized to a pH of about 3 to 7, and it is stated that the gelling agent acts as a compound which allows stabilization of the retinoid and slowly releases it so that it can optimally feed into the keratinocyte layers and dermal cells of the skin.

The present invention provides topical formulations containing retinoic acid which combine cosmetic acceptability with clinical efficacy. In the new formulations, unlike the known compositions, the retinoic acid is entirely dissolved in an essentially aqueous medium, while remaining chemically stable.

The compositions of the present invention for topical application comprise an effective concentration of retinoic acid, and optionally a topically effective, water-soluble antibiotic, dissolved in an aqueous gel based on polyacrylic acid and having a pH in the range of 7 to 8 or, if an antibiotic is present, 6 to 8.

The pH of the composition is preferably adjusted by the addition of a physiologically acceptable amine to a pH such that the polyacrylic acid forms a gel and the retinoic acid, and antibiotic if present, go into solution. Primary, secondary and tertiary alkylamines in which the alkyl groups contain 1 to 4 carbon atoms each are conveniently used and are preferred. The amine should preferably be liquid at ambient temperature. Diethylamine is especially suitable.

The concentration of the retinoic acid in the gel may be in the range from 0.01 to 0.2% w/w, preferably about 0.025 to 0.05%.

The retinoic acid is dissolved in an aqueous gel based on a polyacrylic acid. Such polyacrylic acids are available commercially inter alia under the registered trademark "Carbopol" of which several grades are available, differing principally in their molecular weight. Polyacrylic acids having molecular weights from about 1,000,000 to about 5,000,000 are especially suitable for use in the invention, particularly Carbopol 940 having an approximate molecular weight of 4,000,000. The required concentration of the polyacrylic acid used to form the gels of the invention is low, e.g. 0.2 to 1% in the case of a polyacrylic acid such as Carbopol 940.

The amine is incorporated in the compositions of the invention to achieve the desired pH, to neutralise the carboxylic acid groups of the polyacrylic acid and thereby bring about gel formation, to assist in dissolution of the retinoic acid. It is convenient to measure the amount of amine required by reference to the overall pH of the composition. Sufficient amine is preferably used so that the composition has, when no antibiotic is present, a pH above 7, preferably 7.5-8.0, or when a water-soluble antibiotic is present, 6 to 8.

A preservative, e.g. ethanol, which may be in the form of industrial methylated spirits, is preferably incorporated in the compositions of the invention to inhibit microbiological growth, and to assist dissolution of the retinoic acid. 20 to 40% by weight of the composition may be used. When an antibiotic is present and the pH of the composition is in the range 6 to 7 rather more ethanol is required to maintain dissolution of the retinoic acid than when the composition has a pH in the range 7 to 8. In such circumstances the proportion of ethanol is preferably 30 to 40% by weight. The presence of the ethanol also facilitates evaporation of the gel after application to the skin.

An antioxidant may be included to protect the formulation during manufacture. Propyl gallate is especially suitable. Butylated hydroxytoluene and other suitable antioxidants can also be used.

The new compositions produced in accordance with the invention provide an advantageous way of applying retinoic acid to the skin. The retinoic acid is present in the composition in solution and is absorbed into the skin. The fact that the retinoic acid is present in solution eliminates problems caused by lack of homogeneity which are inherent in compositions containing solid retinoic acid. Furthermore, the compositions evaporate rapidly after application and leave no residue which can cause soiling of clothing or bed linen. The compositions have excellent storage stability.

In the treatment of acne, retinoic acid functions by increasing the rate of epithelialisation within the follicle producing large numbers of loose horny cells which increase the pressure within the sebaceous duct until it becomes sufficiently great to expel the comedo which is blocking the orifice. The drug therefore acts as a comedolytic, allowing the release of blackheads and even whiteheads which are the characteristic lesions associated with the milder grades of acne normally referred to as Grades 1, 2 and 3. However, the more severe types of acne (Grades 4 and 5) are complicated by an additional infective inflammatory reaction producing pustules and even nodules. For these cases, the treatment of choice is the topical application of a suitable antibiotic to destroy the pathogenic anaerobic bacteria, Propionibacterium acnes, which are responsible for the inflammatory reaction. One of the most suitable antibiotics for this purpose is clindamycin, available as both the hydrochloride salt and the phosphate ester, both of which are water-soluble. Other antibiotics which are water-soluble and useful topically in the treatment of acne, e.g. tetracycline or erythromycin in the form of their water-soluble salts, may be incorporated into the formulation instead of clindamycin.

There is, at present, no commercially available topical formulation for the treatment of acne containing, in aqueous solution, both retinoic acid and clindamycin, a combination which could be used in all grades of acne. The present invention makes it possible for the first time to provide a formulation containing both clindamycin phosphate and retinoic acid in an essentially aqueous solution in a gel vehicle, which can be prescribed for all cases of acne, especially those which contain both comedones and inflammatory pustules.

There is some concern regarding the safety of clindamycin, although side effects following topical application have been rare owing to poor absorption through the skin. In this connection, it is important to avoid using clindamycin on irritated skin through which it could be well absorbed. If therefore it were applied in association with retinoic acid and the latter caused an inflammatory irritant reaction of the skin, this could undesirably increase the absorption of the clindamycin. However, the essentially aqueous formulations of retinoic acid of the present invention are less likely to cause irritation than the solid or alcoholic formulations currently available, so that it is now possible, for the first time, to provide a combination containing both retinoic acid and clindamycin in a conveniently used gel formulation.

Clindamycin is normally used topically as the phosphate ester at a concentration equivalent to 1.0% clindamycin. In the presence of retinoic acid, which is known to thin the epidermis, it may be appropriate to reduce the concentration to 0.25 to 0.5%.

The stability of clindamycin falls as the pH of the composition increases from 6 to 8 while the solubility of retinoic acid increases over this pH range. Increasing the proportion of ethanol increases the solubility of retinoic acid but only at the risk of increasing the irritation caused by the composition. The optimum balance of clindamycin stability, retinoic acid solubility, and lack of irritation is achieved at a pH of about 6.5 and 35% by weight ethanol.

The following Examples illustrate the invention.

EXAMPLE 1

Retinoic Acid/Carbopol Gel

|  | % by weight |
|---|---|
| Retinoic Acid | 0.025 |
| Propyl Gallate | 0.05 |
| Industrial Methylated Spirit BP | 20.0 |
| Carbopol 940 | 0.5 |
| Propylene Glycol BP | 5.0 |
| Diethylamine BP | 0.5 (to pH approx. 7.5 to 8.0) |
| Purified Water BP | 73.925 |

The Carbopol is dispersed in the water and the mucilage formed is allowed to stand for one hour with occasional mixing to allow the polymer to swell. The propyl gallate and the retinoic acid are then dissolved in the premixed industrial methylated spirit, propylene glycol and diethylamine. The alcoholic solution is then added slowly with continuous mixing avoiding incorporation of air into the aqueous mucilage until the retinoic acid dissolves in the aqueous solution and the Carbopol becomes sufficiently neutralised to form a stiff clear gel. The gel is then dispensed into tubes or other suitable container.

The propyl gallate may be replaced by butylated hydroxytoluene.

EXAMPLE 2

Retioic Acid/Clindamycin Carbopol Gel

|  | % by weight |
|---|---|
| Retinoic Acid | 0.025 |
| Clindamycin Phosphate | 1.23 (equivalent to 1.0% Clindamycin) |
| Propyl Gallate | 0.05 |
| Industrial Methylated Spirit BP | 35.0 |
| Carbopol 940 | 1.0 |
| Propylene Glycol BP | 5.0 |
| Diethylamine BP | 0.8 (to pH 6.5) |
| Purified Water BP | 56.895 |

The clindamycin phosphate is dissolved in the water and the propylene glycol and half the industrial methylated spirit are then added with continuous stirring. The Carbopol 940 is then dispersed in this aqueous solution and the mucilage formed is allowed to stand for one hour with occasional mixing to allow the polymer to swell.

The propyl gallate and retinoic acid are dissolved in the other half of the industrial methylated spirit and the diethylamine is then added to this alcoholic solution. The alcoholic solution is then added slowly with continuous mixing, avoiding incorporation of air into the aqueous mucilage, until the Carbopol becomes sufficiently neutralised to form a stiff clear gel. The gel is then dispensed into tubes or other suitable container.

The propyl gallate may be replaced by butylated hydroxy toluene.

The compositions of the invention may be applied topically in the treatment of acne in the same way as known retinoic acid-containing compositions but with less risk of irritation, at least equal effectiveness, and greater cosmetic acceptability.

## Claims

1. A composition for topical application comprising an effective concentration of retinoic acid, and optionally a topically effective water-soluble antibiotic, dissolved in an aqueous gel based on polyacrylic acid and having a pH in the range of 7 to 8 or, if an antibiotic is present, 6 to 8.

2. A composition according to claim 1 which comprises a physiologically acceptable, liquid primary, secondary, or tertiary alkylamine in which each alkyl has 1 to 4 carbon atoms in amount such that the pH of the composition is in the stated range.

3. A composition according to claim 2 in which the said alkylamine is diethylamine.

4. A composition according to any one of claims 1 to 3 comprising 0.01 to 0.2% of retinoic acid.

5. A composition according to claim 4 having a pH of about 7.5.

6. A composition according to claim 4 or 5 containing 20 to 40% by weight of ethanol.

7. A composition according to any one of claims 1 to 3 comprising 0.01 to 0.2% of retinoic acid and 0.25 to 1.0% of topically effective antibiotic.

8. A composition according to claim 7 in which the antibiotic is clindamycin phosphate.

9. A composition according to claim 7 or 8 having a pH of about 6.5.

10. A composition according to any one of claims 7 to 9 containing 30 to 40% by weight of ethanol.

11. A composition according to any one of claims 1 to 10 in which the polyacrylic acid has a molecular weight from 1 million to 5 million and the gel contains 0.2 to 1% by weight of the polyacrylic acid.

**European Patent Office**

## EUROPEAN SEARCH REPORT

Application Number

| | DOCUMENTS CONSIDERED TO BE RELEVANT | EP 91308913.2 |
|---|---|---|

| Category | Citation of document with indication, where appropriate, of relevant passages | Relevant to claim | CLASSIFICATION OF THE APPLICATION (Int. Cl.5) |
|---|---|---|---|
| D,P, X | <u>WO - A - 90/14 833</u> (G. BAZZANO) * Claims; examples; page 8, lines 13-29 * -- | 1-4,6, 11 | A 61 K 7/48 A 61 K 31/07 |
| A | <u>US - A - 4 247 547</u> (A.M.MARKS) * Column 2, line 53 - column 5, line 22; examples 4,6 * -- | 1-11 | |
| A | <u>US - A - 4 847 072</u> (D.L.BISSETT et al.) * Column 6, lines 56-59; column 9, lines 17-45 * ---- | 1-11 | |
| | | | TECHNICAL FIELDS SEARCHED (Int. Cl.5) |
| | | | A 61 K 7/00 A 61 K 31/00 |

The present search report has been drawn up for all claims

| Place of search | Date of completion of the search | Examiner |
|---|---|---|
| VIENNA | 06-12-1991 | IRMLER |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&.............................................................................
& : member of the same patent family, corresponding
    document

EPO FORM 1503 03.82 (P0401)